# EUROPEAN PATENT SPECIFICATION

(11) **EP 0 437 373 B1**
(45) Date of publication and mention of the grant of the patent: **29.05.1996**
(21) Application number: 91300201.0
(22) Date of filing: 11.01.1991
(51) Int. Cl.: C12Q 1/26, C12Q 1/32, C12N 9/04, C12N 1/20

(54) **High sensitive assay method of L-carnitine and composition for an assay**
Hochempfindliche Bestimmungsmethode für L-Carnitin und Zusammensetzung für einen Reagenziensatz
Méthode d'essai à sensibilité élevée pour la L-carnitine et composition pour un essai

(30) Priority: 11.01.1990 JP 4063/90; 09.11.1990 JP 305439/90
(43) Date of publication of application: 17.07.1991
(73) Proprietor: ASAHI KASEI KOGYO KABUSHIKI KAISHA, Osaka (JP)
(72) Inventor: Takahashi, Mamoru, Sunto-gun, Shizuoka (JP); Ueda, Shigeru, Tagata-gun, Shizuoka (JP)
(74) Representative: Woods, Geoffrey Corlett

(56) References cited:
- FR-A- 2 398 046
- FR-A- 2 596 865
- FRESENIUS' ZEITSCHRIFT FÜR ANALYTISCHE CHEMIE, vol. 320, no. 3, March 1985, Springer Verlag, Berlin (DE); W. SCHÖPP et al., pp. 285-289/

## Description

This invention relates to a highly sensitive method of assaying L-carnitine and a composition suitable for use therein.

L-carnitine is an essential substance for mediating long-chain fatty acid transport through mitochondrian membranes prior to intracellular β-oxidation. Hence a deficiency in L-carnitine causes fatty acid and related metabolism disorders. It is believed that disorders of the skeletal muscle and cardiac muscle, both of which are high energy consumption tissues depending on cartinine and lacking in carnitine generation, occur from such a deficiency. Previously diseases arising from inborn irregularities of carnitine metabolism have been studied. However, in recent times secondary disorders of carnitine metabolism have become a problem in patients suffering from nephrosis and undergoing dialysis. Carnitine is administered to carnitine deficient patients who have a body muscle or cardiac muscle disease, or to patients undergoing dialysis. Studies on the behaviour of carnitine in disease and therapy have been required. However, a method of assaying carnitine in the clinical field has not been developed.

Known assay methods for carnitine are as follows:
1. L-carnitine and acetyl CoA are treated with carnitine acetyl transferase (CAT), and the thus-liberated CoASH and 5,5'-dithio-bis-2-nitrobenzoate (DTNB) are further reacted to generate thiophenolate ions which are colorimetrically measured (DTNB method). This method is described in J. Biol. Chem., Vol. 238, p. 2509 (1963), J. Lipid Res., Vol. 5, pp. 184-187 (1964) and Clinical Pathology, Vol. 36, N. 11, pp. 1296-1302 (1988).
2. L-carnitine and ¹⁴C- or ³H- labelled acetyl CoA are treated with CAT to generate labelled acetyl-L-carnitine and CoASH, and radio-activity is measured (radioisotope method). This method is described in Clin. Chem. Acta, Vol. 37, pp. 235-243 (1972), J. Lipid Res., Vol. 17, pp. 277-281 (1976), and J. Japan. Nut. Food. Soc., Vol. 41, N. 5, pp. 389-395 (1988).
3. L-carnitine and NAD⁺ are treated with L-carnitine dehydrogenase to generate 3-dehydrocarnitine and NADH, and increased UV absorption of NADH is measured (carnitine dehydrogenase method). This method is described in Eur. J. Biochem., Vol. 6, pp. 196-201 (1968), ibid. Vol. 10, pp. 56-60 (1969), and Fresenius Z. Anal. Chem., Vol. 320, N. 3, pp. 285-289 (1985).
4. L-carnitine and acetyl CoA are treated with CAT to generate CoA which is reacted with n-{p-(2-benzimidazolyl)-phenyl}-malimide (BIPM), and the fluorescent intensity of the resulting CoA-BIPM is measured (fluorescence method). This method is described in Ann. Rep. MHW Institute for Nerve Disease, pp. 315-318 (1986).

In the prior art, the DTNB method and the fluorescence method essentially require a deproteinization treatment in an assay of serum L-carnitine. This creates a complex operation. The radioisotope method has advantages in its sensitivity and specificity. However, special facilities are required for measuring radioactivity. The carnitine dehydrogenase method has a disadvantage due to the small molecular absorption coefficient of NADH, i.e. ε = 6.22 (cm/µmol) at 340 nm. With this method it is difficult to assay serum carnitine in a patient suffering from a carnitine deficient disease [Neurology, 25 : 16-24(1975)]. Moreover generated NADH is consumed by other dehydrogenases in serum such as lactate dehydrogenase. This causes measurement errors.

An assay of L-carnitine, in which formazane generated in an enzymatic reaction with L-carnitine dehydrogenase is quantitatively measured is known (JP-A-1-196550). This method, however, has insufficient sensitivity for the small amount of serum, such as 20µl, which can be collected from a premature infant as compared with the relatively large amount of serum which can be obtained from an adult.

Under these circumstances, it has been desired to develop an advantageous method for assaying L-carnitine which does not require complex treatment such as deproteinization and specific facilities and which can measure trace amounts of serum L-carnitine of a premature infant.

FR-2,596,865 describes an assay method for carnitine in which the amount of carnitine in a sample is assayed by adding NAD⁺ in the presence of a carnitine dehydrogenase and measuring the amount of NADH produced.

We have studied a reaction system using L-carnitine dehydrogenase (EC 1.1.1.108). We have found that, in a reversible cycling reaction in which dehydrocarnitine is generated from an L-carnitine substrate with a coenzyme of the NAD group, together with a trace amount of a NADH group coenzyme, a linear increase in the amount of NADH group generated was observed over time. Furthermore the rate of generation thereof was in proportion to the amount of L-carnitine or dehydrocarnitine.

We have further found that in said enzymatic cycling reaction, a coenzyme of the thionicotinamide adenine dinucleotide group (hereinafter designated as thio-NAD group) or reduced form thio-NAD group (hereinafter designated as thio-NADH group) is used with the coenzymes of the NAD group or NADH group. The change in the amounts of any of the coenzymes can be measured and depends upon a difference in a maximum absorption of the NADH group at approx. 340 nm and that of the thio-NADH group at approx. 400 nm. Thus the amount of L-carnitine or dehydrocarnitine can precisely be measured.

The present invention provides a highly sensitive method of assaying L-carnitine which comprises reacting a specimen comprising the L-carnitine to be assayed with reagents comprising:
1) L-carnitine dehydrogenase which has coenzymes of the thionicotinamide adenine dinucleotide group (hereinafter designated as the thio-NAD group) and nicotinamide adenine dinucleotide group (hereinafter designated as the NAD group) and which catalyses a reversible reaction forming essentially dehydrocarnitine from L-carnitine;
2) A₁; and
3) B₁;
   to perform the cycling reaction: 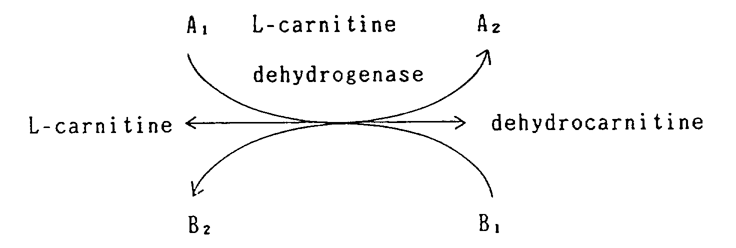 wherein:
   A₁ is the thio-NAD group or the NAD group;
   A₂ is a reduced form of A₁;
   when A₁ is the thio-NAD group, B₁ is a reduced NAD group and when A₁ is the NAD group, B₁ is a reduced thio-NAD group, and B₂ is an oxidized form of B₁;
   and measuring the amount of A₂ generated or B₁ consumed.

The present invention also provides a composition suitable for assaying L-carnitine which comprises components 1-3 as defined above.

Figure 1 is a rate assay graph for L-carnitine at 400 nm as in Example 1.

Figure 2 is a rate assay graph for serum at 400 nm as in Example 2.

In the present invention any L-carnitine dehydrogenase can be used so long as it has the above properties. Examples of L-carnitine dehydrogenases are those produced by the following microorganisms:
Pseudomonas aeruginosa A 7244 (NCTC) [Eur. J. Biochem., Vol. 6, pp. 196-201 (1968), ibid., Vol. 10, pp. 56-60 (1969)] ;
Pseudomonas putida IFP 206 [Arch. Microbiol., Vol. 116, pp. 213-220 (1978), Biochem. Biophys. Acta, Vol. 957, pp. 335-339 (1988)] ;
Pseudomonas putida ATCC 17633 [Fresenius' Z. Anal. Chem., Vol. 320, pp. 285-289 (1985)];
Xanthomonas translucens IFO 13558 [Agr. Biol. Chem., Vol. 52, pp. 851-852 (1988)]; and
Alcaligenes sp. No. 981 FERM BP-2570 [Product of Toyo Jozo Co., JP-A-1-267919].

Among these, L-carnitine dehydrogenase originated from Alcaligenes sp. No. 981 is preferable because of its stability in a buffer solution.

L-carnitine dehydrogenase originated from Alcaligenes sp. No. 981 is produced, for example, by a microorganism of the genus Alcaligenes sp. No. 981 FERM BP-2570, isolated from a soil sample from a potato field in Gojo-shi, Nara prefecture, Japan.

The taxonomical properties of this strain are:

### A. Morphological properties:

Observations on a nutrient agar medium, cultured for 18-24 hours at 28-30°C, are as follows.

Round edge with straight or slightly curved bacillus and single or double linked somewhat short chain. No formation of spores. Sizes are 0.4-0.6 x 1.2-2.5µm. Peritrichal movement. No polymorphism.

### B. Growth on various media:

Observations on various media, cultured for 18-24 hours at 28-30 °C, are as follows.
1. Nutrient agar slant medium:
   Good growth with filiform.
   Wettish with luminescence. Ocherous but no formation of soluble pigment.
2. Nutrient agar plate medium :
   Round, convex and whole round colonies. Smooth wettis surface. Ocherous or pale ocherous.
   No formation of soluble pigment.
3. Liquid medium (Aqueous peptone):
   Good growth with uniform turbidity. Formation of pellicle at long term (over 40 hours) culture.
4. BCP milk medium:
   Alkaline after 4-5 days.

### C. Physiological properties (+ = positive, ( +)= weekly positive, - = negative )

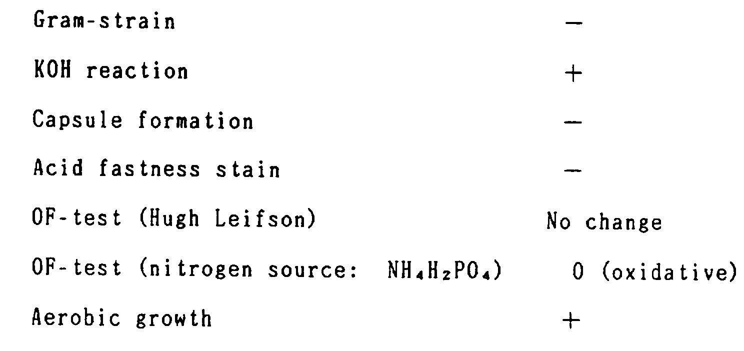

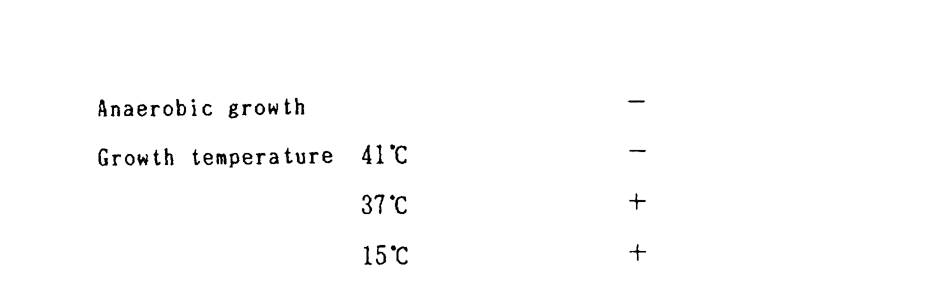

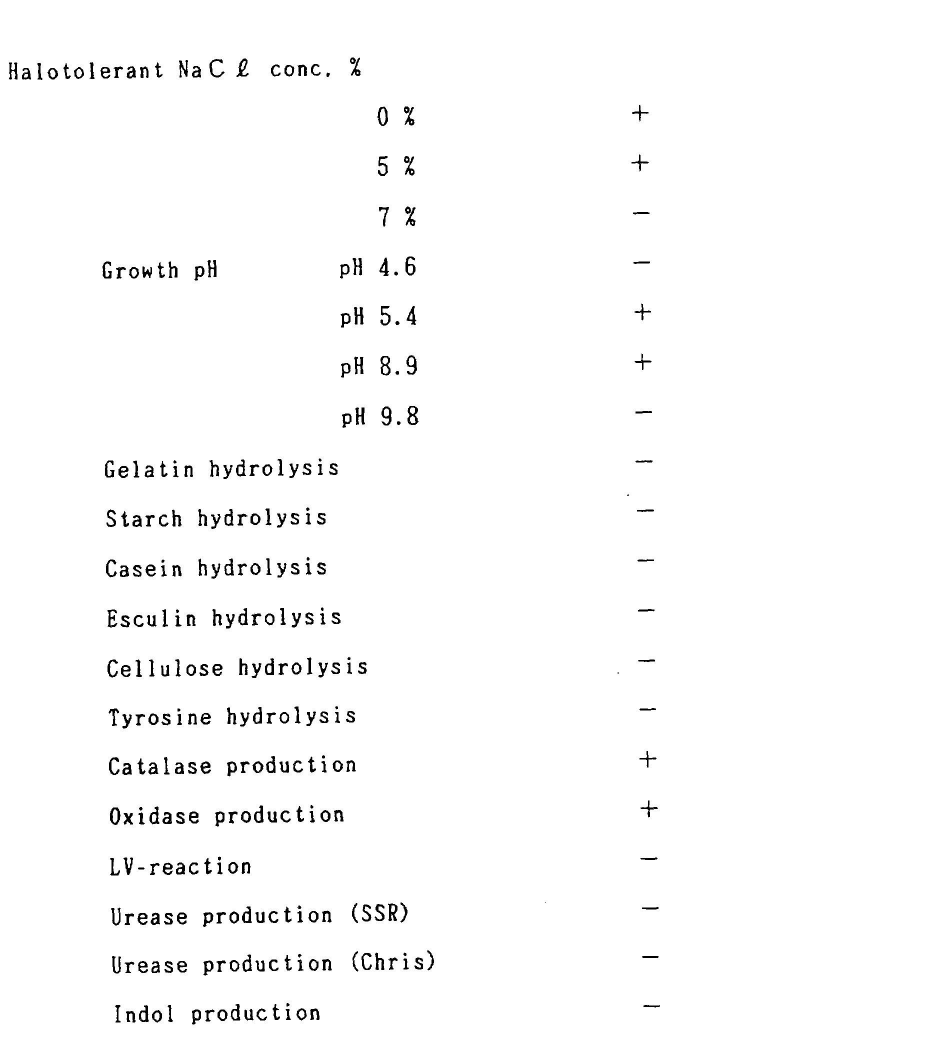

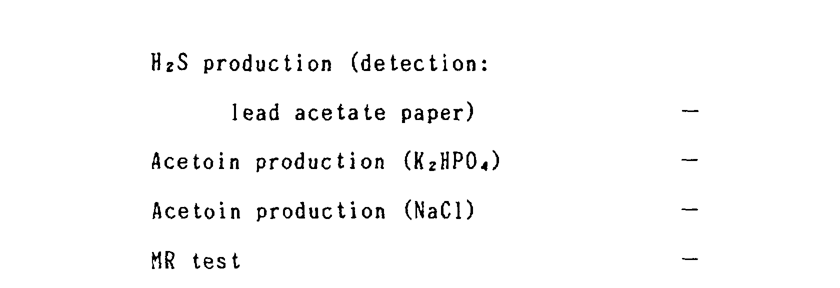

| Nitrate reduction | |
|---|---|
| Gas detection | + |
| N0₂ - detection | - |
| N0₃ - detection | - |

| Utilization on Simmons medium | |
|---|---|
| Citrate | + |
| Malate | + |
| Maleate | - |
| Malonate | (+) |
| Propionate | - |
| Gluconate | - |
| Succinate | + |

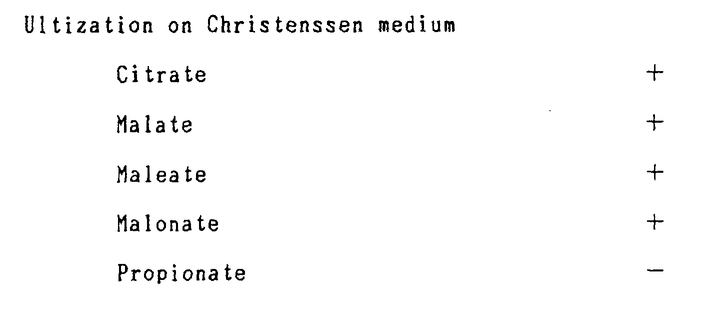

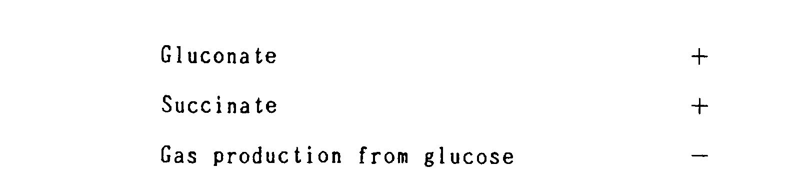

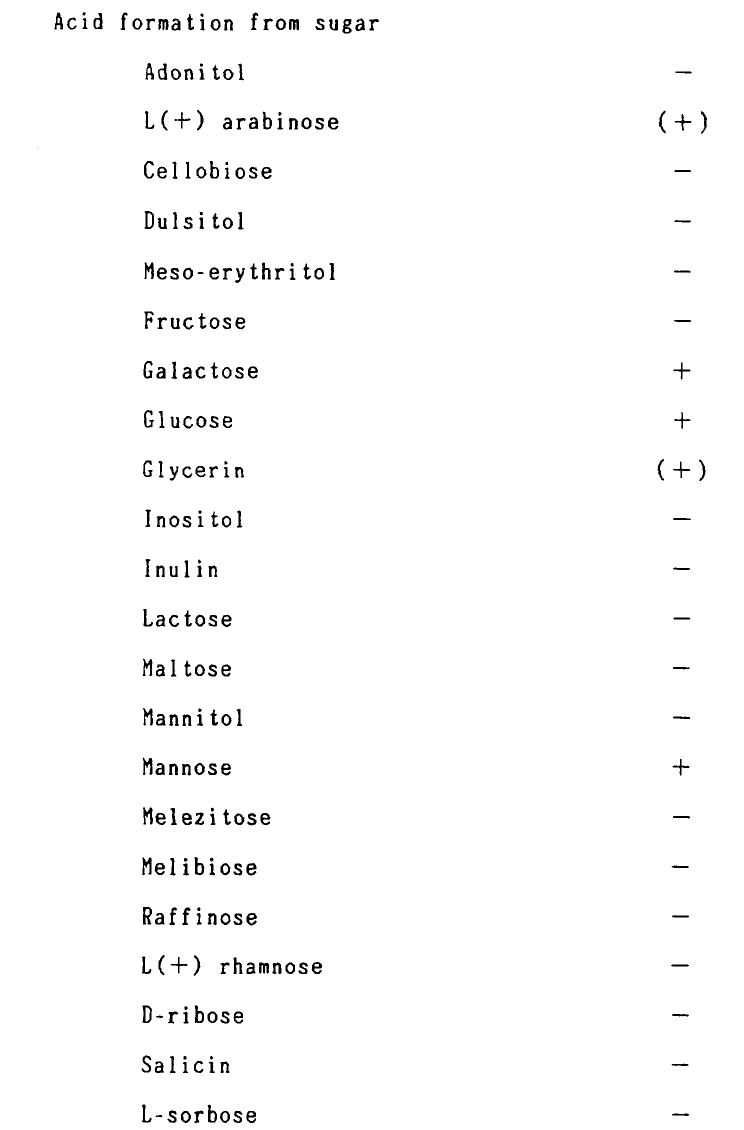

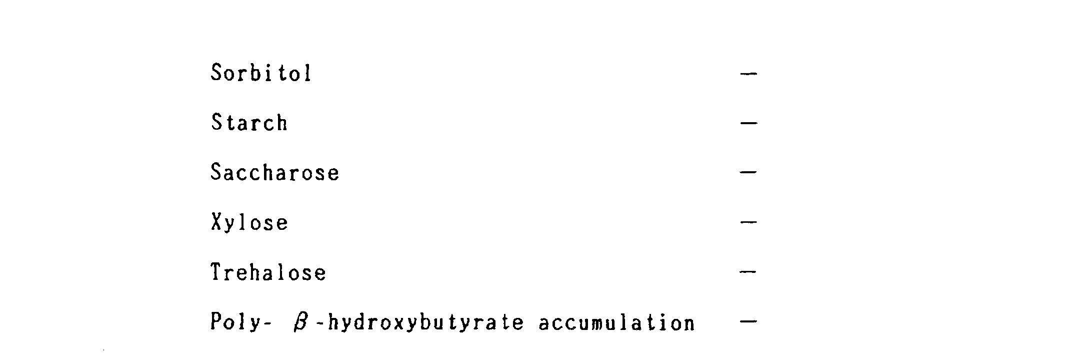

### D. Utilization of carbon sources:

Test medium: liquid medium (pH 7.0) containing carbon source 5 g, NaCl 5 g, MgSO₄·7H₂O 0.2 g, NH₄H₂PO₄ 1.0 g and distilled water 1 ℓ it. Results are as follows:

| | |
|---|---|
| Glucose | + |
| L(+) arabinose | - |
| Fructose | + |
| Mannitol | - |
| Mannose | + |
| Gluconate | + |
| Acetate | + |
| Adipate | - |
| Pimerate | + |
| Suberate | + |
| Tartrate | + |

According to the above taxonomical properties, the microorganism displays the specific characteristics of Gram negative bacillus, namely, it is peritrichal in movement, is catalase positive and oxidase positive, does not produce acid from glucose in Hugh-Leifson medium containing peptone, and promotes oxidative decomposition of glucose and acid formation. It displays no spore formation nor polymorphism, and is aerobic.

Among Gram-negative bacillus, there are three microorganism genera which are peritrichal in movement, namely Alcaligenes, Chromobacterium and Flavobacterium. Chromobacterium produces violet colored pigment, and Flavobacterium produces yellow colored pigment; however, the present strain does not produce pigment. Hence the present strain belongs to the genus Alcaligenes.

The taxonomic properties of Alcaligenes in comparison with those of the present strain according to Bergey's Manual of Systematic Bacteriology, Vol. 1 (1984), are illustrated by comparing Alcaligenes faecalis (hereinafter designated as F), Alcaligenes denitrificans subsp. denitrificans (hereinafter designated as D) and Alcaligenes denitrificans subsp. xylosoxidans (hereinafter designated as X), as follows:
+ = positive probability over 90%.
- = negative probability over 90%.
d = not identified as + or -.

| | F | D | X | The present Strain |
|---|---|---|---|---|
| Oxidase production | + | + | + | + |
| Nitrate reduction | - | + | + | + |
| Nitrite reduction | + | + | + | + |
| Gelatin hydrolysis | - | - | - | - |

| Acid formation in OF-medium | | | | |
|---|---|---|---|---|
| Xylose | - | - | + | - |
| Glucose | - | - | + | - |

| Acid formation in peptone-free medium | | | | |
|---|---|---|---|---|
| Xylose | | | + | - |
| Glucose | | | + | + |

| Utilization of carbon sources | | | | |
|---|---|---|---|---|
| Glucose | - | - | + | + |
| L(+) arabinose | - | - | - | - |
| Fructose | - | - | d | + |
| Mannitol | - | - | - | - |
| Mannose | - | - | d | + |
| Gluconate | - | + | + | + |
| Acetate | + | + | + | + |

According to the above comparison, the present strain No. 981 has many identical properties with Alcaligenes denitrificans subsp. xylosoxidans but has specific differences as to acid formation in OF-medium and acid formation from xylose. Accordingly, the present strain has been designated Alcaligenes sp. No. 981 and has been deposited at the Fermentation Research Institute and assigned No. FERM BP-2570.

In the enzymatic reaction hereinbefore illustrated, A₁ or B₂ is a coenzyme of the thio-NAD group or NAD group. Examples of the NAD group are nicotinamide adenine dinucleotido (NAD), acetylpyridine adenine dinucleotide (acetyl NAD), acetylpyridine hypoxanthine dinucleotide and nicotinamide hypoxanthine dinucleotide (deamino NAD). Examples of the thio-NAD group are thionicotinamide adenine dinucleotide (thio-NAD) and thionicotinamide hypoxanthine dinucleotide.

In the present invention, when A₁ is the thio-NAD group, B₁ is the NAD group, and when A₁ is the NAD group, B₁ is the thio-NADH group. Hence at least one should be a thio-type coenzyme.

The amounts of A₁ and B₁ are in excess as compared with that of the L-carnitine. The amounts are essentially in excess as compared with the Km-value of carnitine dehydrogenase for A₁ and B₁. Specifically a 20-10,000 molar excess over the amount of carnitine is preferable.

In a composition for assay of L-carnitine of the present invention, the concentration of A₁ and B₁ is generally 0.02-100 mM, preferably 0.05-30 mM, and the concentration of L-carnitine dehydrogenase is generally 5-1000 U/ml, preferably 10-150 U/ml, or more.

L-carnitine dehydrogenase used in the assay composition of the present invention is an enzyme having reactivity with an L-carnitine substrate together with coenzymes of the NAD group and thio-NAD group. It can be identified by using said coenzymes and substrate. L-carnitine dehydrogenase produced by Alcaligenes sp. No. 981 (product of Toyo Jozo) has a relative activity of approx. 15% when coenzyme thio-NAD is used as compared with NAD. The Km-values for L-carnitine, NAD and thio-NAD under the same conditions are 9.3 mM, 0.14 mM and 0.40 mM, respectively.

In the reaction medium composition, two coenzymes are selected by considering their relative activity with L-carnitine dehydrogenase. Thereafter the pH condition, considering the optimum pH of the forward reaction and the reverse reaction, is adjusted such that the ratio of the forward reaction rate to the reverse reaction rate is 1:1.

In the present invention, L-carnitine dehydrogenase from a single origin or a plurality of origins can be used.

L-carnitine in a specimen can be assayed by- adding 0.001-0.5 ml of the specimen to the assay composition containing the above components 1-3, carrying out a reaction at 37°C, and then measuring the amount of generated A₂ or consumed B₁ for a duration between two points after starting the reaction. For example the measurement can be carried out for one minute between 3 minutes and 4 minutes after starting, or for 5 minutes between 3 minutes and 8 minutes after starting the reaction. The measurement may be carried out by determining the changes of absorption at each optical absorption. Alternatively, the enzymatic reaction is stopped after a time from starting the reaction, for example after 10 minutes. The changes of absorption value can then be measured. For example, when A₂ is thio-NADH and B₁ is NADH, the amount of A₂ generated is measured by an increase of absorption at 400 nm [molecular absorption coefficient: 11,200 M⁻¹cm⁻¹ (Methods in Enzymolgy. Vol. 55, p. 261 (1979)] or the amount of B₁ consumed is meausred by a decrease of absorption at 340 nm (molecular absorption coefficient: 6220 M⁻¹ cm⁻¹). The thus obtained value is then compared with the value of a known concentration of authentic L-carnitine. Thus the concentration of L-carnitine in a specimen can be meausred in real-time.

According to the assay method of the present invention, since L-carnitine per se in a specimen is introduced into an enzymatic cycling reaction, coexisting substances in the specimen do not affect the result. Hence a control measurement of a specimen not containing L-carnitine is not required. Thus a simple assay system by a rate assay can be achieved.

Measuring the amount of A₂ or B₁ by absorbancy can be replaced by other known enzymatic methods.

In the assay method of the present invention, not only free L-carnitine but also liberated L-carnitine from hydrolysis of an acyl carnitine such as acetyl carnitine can also be assayed. L-carnitine per se in a specimen can directly be assayed by the assay method of the present invention without hydrolysis of the specimen. Thereafter the total amount of L-carnitine and acylcarnitine can be assayed after hydrolysing the specimen. The amount of acylcarnitine in the specimen can be obtained by deducting the amount of L-carnitine in the specimen without hydrolysis from the total amount of L-carnitine after hydrolysing acylcarnitine in the specimen.

Furthermore, a substrate in an enzymatic reaction system with generation or consumption of L-carnitine, or an enzymatic activity thereof, can also be assayed. Examples of these enzymatic systems are:

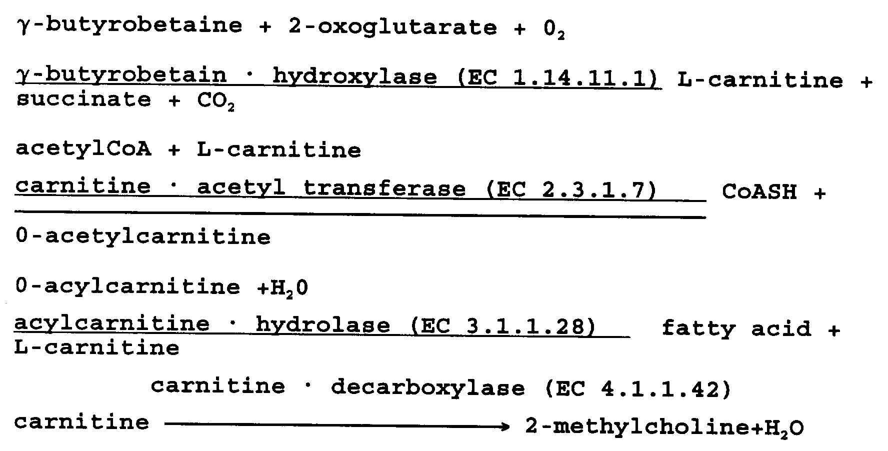

In these systems, measurement of substrate or enzymatic activity can be achieved by the assay method of the present invention.

The present invention has advantages in that no error of measurement occurs due to the use of coenzymes having different absorptions in their reduced forms. Free L-carnitine, total carnitine and acylcarnitine can also be assayed precisely and rapidly, even for a small amount of specimen.

The following Examples further illustrate the present invention.

### Ref. Example 1

(i) Culturing Alcaligenes sp. No. 981:

| | |
|---|---|
| DL-carnitine hydrochloride (Sigma Chem., Co.) | 3.0 % |
| KH₂PO₄ | 0.2 % |
| K₂HPO₄ | 0.4 % |
| MgSO₄ · 7H₂O | 0.05 % |
| FeSO₄ · 7H₂O | 0.002% |
| MnSO₄ · nH₂O | 0.001% |
| pH 7.0 | |

100 ml of a liquid medium comprising the above composition was sterilized in a 500 ml Erlenmeyer flask at 120°C for 20 minutes. One loopful of Alcaligenes sp. No. 981 was inoculated into the medium and the medium was cultured at 28°C with stirring at 120 rpm for 40 hours to obtain the cultured mass (95 ml) (enzyme activity: 1.2 U/ml).
(ii) DL-carnitine hydrochloride (Sigma Chem. 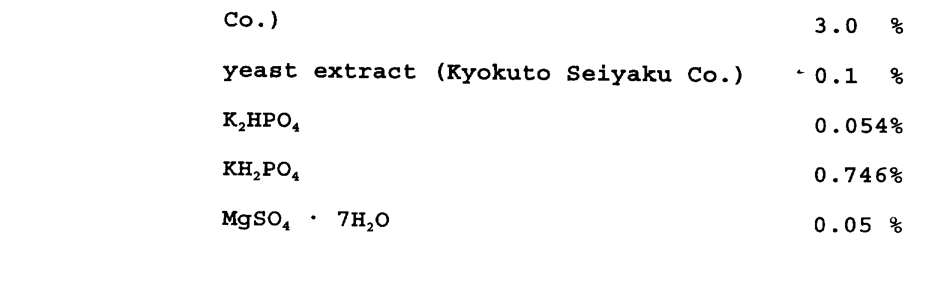 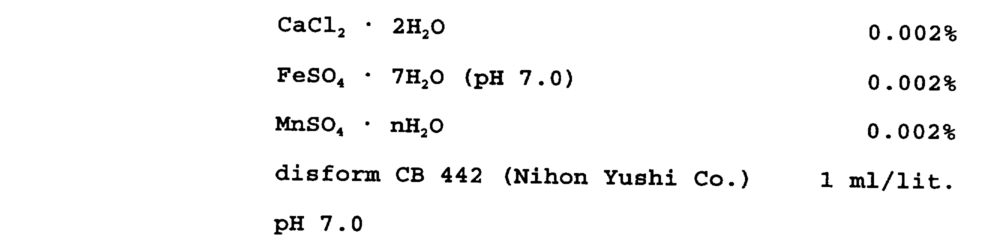 20 l of a liquid medium comprising the above composition was sterilized in a 30 l jar fermenter by heating. 90 ml of the precultured seed culture obtained in step (i) above was inoculated therein and the mixture was cultured at 28°C, with aeration of 20 l/min, inner pressure 0.4 kg/cm, and agitation at 200 rpm for 27 hours to obtain the cultured mss (19 l).(enzyme activity: 3.0 U/ml).

### Ref. Example 2

Purification of enzyme:

Bacterial cells collected by centrifugation from the cultured broth (19 l) obtained in Ref. Example 1, Culture (ii), were suspended in 40 mM Tris-HCl buffer (pH 8.0, 5 l) containing 0.1% lysozyme and 15 ml EDTA · 2 Na and solubilized at 37°C for 1 hour; then the mixture was centrifuged to remove precipitate and to obtain a supernatant solution (4500 ml) (activity: 10.3 U/ml). 1100 g ammonium sulfate was added to the supernatant solution, which was mixed well by stirring and then centrifuged to separate the precipitate. An additional 700 g ammonium sulfate was then added to the supernatant solution to dissolve the precipitate, and the solutibn was centrifuged to obtain a new precipitate. The new precipitate was dissolved in 40 mM Tri-HCl buffer (pH 8.0, 500 ml)(specific activity 84.1 U/ml), and the resultant solution was dialysed against 40 mM Tris-HCl buffer (pH 8.0, 10 lit.). The dialyzed enzyme solution was charged on a column of DEAE-Sepharose CL-6B (Pharmacia Co.) (200 ml) which was buffered with 40 mM Tris-HCl buffer (pH 8.0), washed with 40 mM Tris-HCl buffer containing 0.1 M KCI, (pH 8.0, 1 lit.) and eluted with 40 mM Tris-HCl buffer containing 0.3 M KCI (pH 8.0) to obtain an enzyme solution (300 ml, specific activity 120.5 U/ml). The enzyme solution was dialysed against 40 mM Tris-HCl buffer (pH 8.0, 10 lit.). The Dialysed enzyme solution was charged on a column of hydroxylapatite (KOKEN Co., 100 ml), which was buffered with 40 mM Tris-HCl buffer, washed with 40 mM Tris-HCl buffer, washed with 40 mM Tris-HCl buffer (pH 8.0, 200 ml), then eluted with 2 mM phosphate buffer (pH 7.0, 100 ml) to obtain enzyme solution (100 ml, specific activity 331 U/ml). The thus obtained enzyme solution was dialysed against 20 mM phosphate buffer (pH 7.5, 5 lit.) to obtain 95 ml of an enzyme solution having a specific activity of 331 U/ml. The yield was 67.8 %.

The purified L-carnitine dehydrogenase was found to have an NADH oxidase activity of less than 0.0001 U/ml.

The L-carnitine dehydrogenase thus obtained has the following properties:

### 1. Enzyme action:

The enzyme catalyses at least a reaction of L-carnitine and NAD ⁺ to generate 3-dehydrocarnitine and NADH, as shown below.

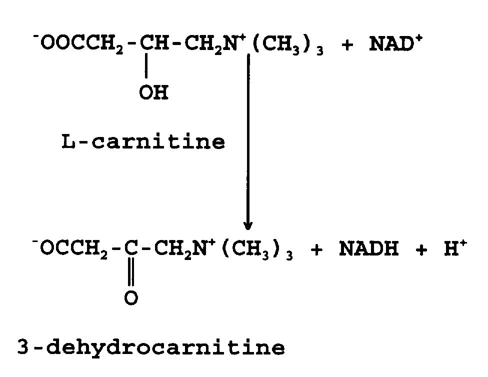

### 2. Substrate specificity:

| | |
|---|---|
| L-carnitine | 100% |
| Choline | 0 |
| Glycinebetaine | 0 |
| Glucose | 0 |
| Lysine | 0 |

### 3. Molecular weight:

| |
|---|
| 51000 ± 6000 |

Measured by TSK-gel G3000 SW (Toso Co., 0.75 x 60 cm)
Elution: 0.1 M phosphate buffer (pH 7.0) containing 0.2 M NaCl.
Standard: following molecular markers (Oriental yeast Co.) are used.

| | |
|---|---|
| M.W. 12,400 | Cytochrome C |
| M.W. 32,000 | adenylate kinase |
| M.W. 67,000 | enolase |
| M.W. 142,000 | lactase dehydrogenase |
| M.W. 290,000 | glutamate dehydrogenase |

### 4. Isoelectric point:

| |
|---|
| pH 5.3 ± 0.6 |

Measured by electrofocussing using carrier ampholite at 4°C, 700V, for 40 hours. The activity of a fraction of each enzyme is measured.

### 5. Km-value: 0.141 mM (NAD⁺), 9.3 mM (L-carnitine)

Km-value for NAD⁺ is measured in various concentrations of NAD⁺ in a reaction mixture of:

| | | |
|---|---|---|
| 100 mM | Tris-HCl | buffer (pH 9.0) |
| 5 U | diaphorase | (Toyo Jozo Co.) |
| 0.025% | NBT | (Wako Pure Chem. Co.) |
| 1% | polyoxyethylene (20) sorbitan monooleate 80 | (Wako Pure Chem., Co) and |
| 50 mM | L-carnitine. | |

In the reaction mixture, 50 mM L-carnitine is replaced by 1 mM NAD⁺, and concentration of L-carnitine is varied to measure the Km-value of L-carnitine. The results are as shown above.

### 6. Heat stability:

The enzyme, dissolved in 20 mM Tris-HCl buffer (pH 8.0), to produce a 1.00 U/ml solution, is incubated for one hour at various temperatures, and the residual activity is measured.

From the results it can be seen that the enzyme is stable up to 45°C.

### 7. Optimum temperature:

The enzyme activity is measured at 35, 40, 45, 50, 55 and 60°C, respectively, in 100 mM Tris-HCl buffer (pH 9.0) according to the assay method illustrated hereinafter. The reaction was stopped in each case after 10 mins. incubation by adding 0.1 N HCl (2 ml), whereupon the optical absorption at 550 nm was measured. The enzyme shows maximum activity at 50°C.

### 8. pH - stability:

The residual activity of the enzyme (1 U/ml, 40 mM buffer solution) is measured in various buffer solutions, i.e. acetate buffer, pH 5.6-6.0; phosphate buffer, pH 6.0-8.0; Tris-HCl buffer, pH 8.0-9.0 and glycine-NaOH buffer, pH 9.0-10, after heating at 45°C for 30 mins. The enzyme is stable at pH 8.0-9.0 with a residual activity of over 95%.

### 9. Optimum pH:

In an assay method for enzyme activity as illustrated hereinafter, 100 mM Tris-HCl buffer in the reaction mixture is replaced by 100 mM phosphate buffer (pH 6.5-7.5), 100 mM Tris-HCl buffer (pH 8.0-9.0) and 100 mM glycine-NaOH buffer (pH 9.0-10.0), and incubated at 37°C for 10 mins. The reaction was stopped in each case by adding 0.1 N HCl (2 ml), whereupon the absorption at 550 nm was measured.

A maximum activity is observed at approx. pH 9.0.

### 10. Long term stability in aqueous solution:

Stability of L-carnitine dehydrogenase with 0.05 mM NAD added is measured in 50 mM Tris-HCl buffer (pH 9.0, 10 U/ml) at 5°C after two weeks storage.

L-carnitine dehydrogenase has a residual activity of 96% after one week and 82% after two weeks, thus showing superior stability.

### 11. Assay method of L-carnitine dehydrogenase activity:

### (1) Reaction mixture:

| | | |
|---|---|---|
| 50 mM | Tris-HCl buffer (pH 9.0) | |
| 1 mM | NAD⁺ | |
| 5 U | Diaphorase | (Toyo Jozo Co.) |
| 0.05% | NBT | (Wako Pure Chem. Co.) |
| 100 mM | KCl | |
| 0.5% | polyoxyethylene (20) sorbitan monooleate (Wako Pure Chem. Co.) | |
| 100 mM | L-carnitine | (Sigma Chem. Co.) |

### (2) Enzyme assay:

The above reaction mixture (1 ml) is incubated in a small test tube at 37°C for 5 mins. Dilute enzyme solution (0.02 ml) is added and stirred to initiate the reaction. After exactly 10 mins., 0.1 N HCl (2.0 ml) was added and stirred to stop the reaction. Absorption at 550 nm (A₅₅₀ nm) is measured to obtain absorption A₁. The assay was repeated using the above reaction mixture except that L-carnitine was not included. The mixture is also treated in the same manner as described above and its absorption Ao was measured.

### (3) Calculation of enzyme activity:

$\text{U/ml =} \frac{{\text{(A}}_{\text{1}} \text{- Ao)}}{\text{21.7}} \text{x} \frac{\text{1}}{\text{10}} \text{x} \frac{\text{3.02}}{\text{0.02}} \text{x Z}$ wherein
21.7: molecular absorption coefficiency cm/µmol
Z : dilution ratio

### Example 1

### Reagent:

| | |
|---|---|
| 100 mM | Tris-HCl buffer (pH 9.5) |
| 5 mM | Thio-NAD (Sankyo Co.) |
| 0.2 mM | NADH (Oriental Yeast Co.) |
| 92 U/ml | L-carnitine dehydrogenase (obtained from Ref. Example 2) |

### Operation:

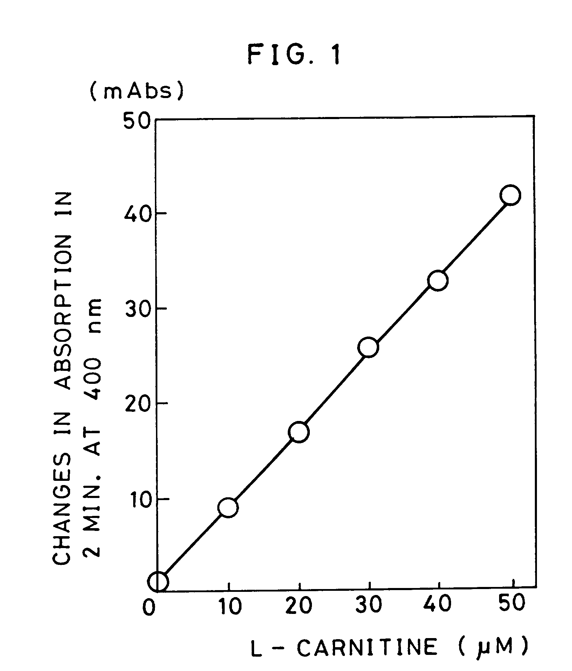

The above reagent (1 ml) was put into cuvettes, L-carnitine solution (0.05 ml) (0, 10, 20, 30, 40 and 50 µM, respectively) was added therein, and the reaction was started at 37°C. After incubation was started, the difference in absorbancy at 3 mins. and 5 mins. was measured. The result is shown in Fig. 1. A linear relation between the amount of L-carnitine and the change in absorption was observed.

### Example 2

### Reagent:

| | |
|---|---|
| 40 mM | Glycine · NaOH buffer (pH 10.0) |
| 5 mM | Thio-NAD (Sankyo Co.) |
| 0.5 mM | NADH (Oriental Yeast Co.) |
| 0.5% | polyoxyethylene (20) sorbitan monooleate (Wako Pure Chem. Co.) |
| 120 U/ml | L-carnitine dehydrogenase (obtained from Ref. Example 2) |
| 2 mM | Oxamic acid |

### Operation:

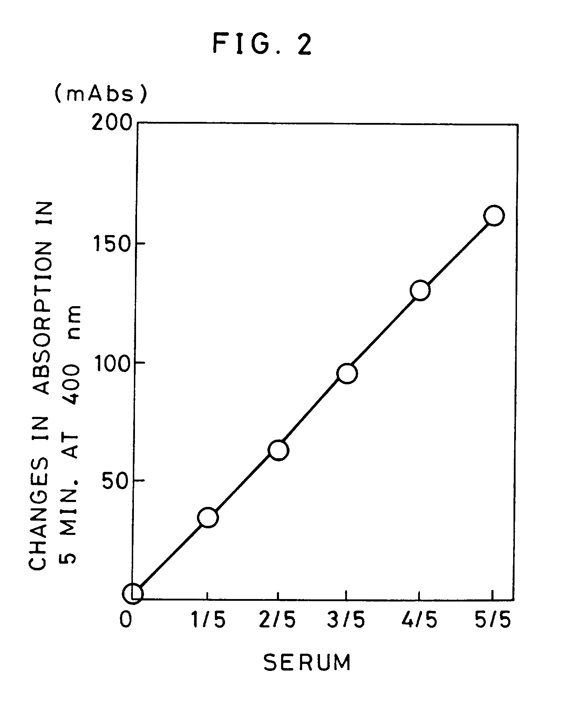

The above reagent (1 ml) was put into a cuvette. A five-fold dilution of normal serum (each 50 µl) was added therein, and incubated at 37°C. After the reaction was started, absorbancy at 400 nm was measured at 1 min. and 6 min. Differences in absorbancy at 1 min. and 6 min. is shown in Fig. 2.

50 µM L-carnitine solution (50 µl) was also treated in the same manner as described above. Then the amount of L-carnitine in the normal serum was calculated and was determined to be 54.3 µM.

### Example 3

### Reagent:

| | |
|---|---|
| 40 mM | Glycine · NaOH buffer (pH 10.0) |
| 5 mM | Thio-NAD (Sankyo Co) |
| 0.5 mM | NADH (Oriental Yeast Co.) |
| 0.5% | Polyoxyethylene (20) sorbitan monooleate (Wako Pure Chem. Co.) |
| 120 U/ml | L-carnitine dehydrogenase (obtained from Ref. Example 2) |
| 2 mM | Oxamic acid |

### Operation:

The above reagent (1 ml) was put into cuvettes. Specimen (50 µl), to which L-carnitine was added to normal serum in concentrations of 10, 20 and 50 µM, was treated in the same manner as described in Example 2. The results are shown in Table 1, where the yield is observed as 97.0-102.0%.

**Table 1**

| Amount added (µM) | Observed (µM) | Difference (µM) | Yield (%) |
|---|---|---|---|
| 0 | 54.3 | - | - |
| 10 | 64.5 | 10.2 | 102.0 |
| 20 | 74.2 | 19.9 | 99.5 |
| 50 | 103.3 | 48.5 | 97.0 |

### Example 4

### Reagent:

| | |
|---|---|
| 40 mM | Glycine · NaOH buffer (pH 10.0) |
| 5 mM | Thio-NAD (Sankyo Co.) |
| 0.2 mM | NADH (Oriental Yeast Co.) |
| 0.5% | Polyoxyethylene (20) sorbitan monooleate (Wako Pure Chem. Co.) |
| 100 U/ml | L-carnitine dehydrogenase (obtained from Ref. Example 2) |
| 2 mM | Oxamic acid |

### Operation:

2 mM Tris-HCl buffer (0.025 ml) and 1 N KOH (0.025 ml) were added to serum (0.05 ml). Acylcarnitine in serum was hydrolysed by incubating at 37°C for one hour. Thereafter the reaction mixture was neutralized by adding 2.5 N HCl (0.05 ml) to prepare a specimen for total carnitine assay. A mixture of serum (0.05 ml) and physiological saline (0.1 ml) was prepared as a specimen for free carnitine assay.

Each above reagent (1 ml) was put into cuvettes, and a specimen hereinabove prepared (each 0.05 ml) was added to each cuvette, then incubated at 37°C. After the reaction was started, the difference at 1 min. and 6 min. in absorption at 400 nm was obtained.

L-carnitine with a known concentration was treated in the same manner as above, and the amount of total carnitine and of free carnitine were calculated from the observed value. The amount of acylcarnitine was determined as the difference in a mounts of total and free carnitine.

Table 2, shows the results obtained from three different sera.

**Table 2**

| | Total carnitine (µM) | Free carnitine (µM) | Acylcarnitine (µM) |
|---|---|---|---|
| Serum 1 | 84.5 | 35.3 | 49.2 |
| Serum 2 | 50.0 | 26.3 | 23.7 |
| Serum 3 | 89.5 | 43.2 | 46.3 |

## Claims

1. A method of assaying L-carnitine which comprises reacting a specimen comprising the L-carnitine to be assayed with reagents comprising:
1) L-carnitine dehydrogenase which has coenzymes of the thionicotinamide adenine dinucleotide group (hereinafter designated as the thio-NAD group) and nicotinamide adenine dinucleotide group (hereinafter designated as the NAD group) and which catalyses a reversible reaction forming essentially dehydrocarnitine from L-carnitine;
2) A₁; and
3) B₁;
to perform the cycling reaction: 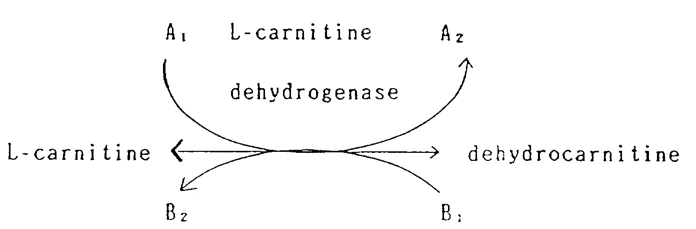 wherein:
A₁ is the thio-NAD group or the NAD group;
A₂ is a reduced form of A₁;
when A₁ is the thio-NAD group, B₁ is a reduced NAD group and when A₁ is the NAD group, B₁ is a reduced thio-NAD group, and
B₂ is an oxidized form of B₁;
and measuring the amount of A₂ generated or B₁ consumed.

2. A method according to claim 1 wherein the L-carnitine dehydrogenase has been obtained from the genus Alcaligenes.

3. A method according to claim 2 wherein the L-carnitine dehydrogenase has been obtained from Alcaligenes sp. No. 981 FERN BP-2570.

4. A method according to any one of the preceding claims wherein the NAD group is nicotinamide adenine dinucleotide (NAD), acetylpyridine adenine dinucleotide (acetyl NAD), acetylpyridine hypoxanthine dinucleotide or nicotinamide hypoxanthine dinucleotide (deamino-NAD).

5. A method according to any one of the preceding claims wherein the thio-NAD group is thionicotinamide adenine dinucleotide (thio-NAD) or thionicotinamide hypoxanthine dinucleotide.

6. A composition suitable for assaying L-carnitine which comprises components 1 - 3 as defined in any one of the preceding claims.

## Patentansprüche

1. Verfahren zum Bestimmen von L-Karnitin, bei dem man eine Probe mit L-Karnitin, das zu bestimmen ist, mit Reagenzien umsetzt, die umfassen:
1) L-Karnitin-dehydrogenase, die mit Coenzymen der Thionicotinamid-adenin-dinucleotid-Gruppe (nachstehend: Thio-NAD-Gruppe) und Nicotinamid-adenin-dinucleotid-Gruppe (nachstehend NAD-Grupe) vorliegt und eine reversible Reaktion unter Bildung von im wesentlichen Dehydrokarnitin aus L-Karnitin katalysiert;
2) A₁; und
3) B₁;
und die folgende zyklische Reaktion durchführt: 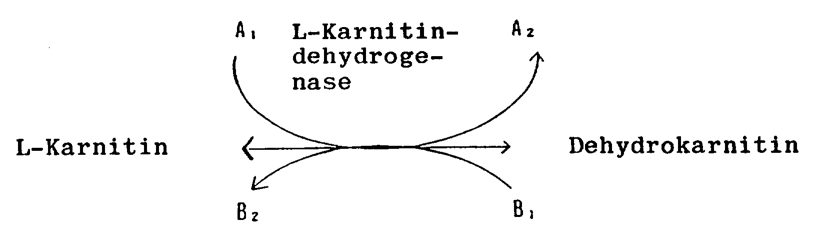 worin
A₁ die Thio-NAD-Gruppe oder die NAD-Gruppe ist;
A₂ eine reduzierte Form von A₁ ist;
für A₁ als Thio-NAD-Gruppe B₁ eine reduzierte NAD-Gruppe ist und
für A₁ als NAD-Gruppe B₁ eine reduzierte Thio-NAD-Gruppe ist und
B₂ eine oxidierte Form von B₁ ist; und
die Menge an gebildetem A₂ oder an verbrauchtem B₁ mißt.

2. Verfahren nach Anspruch 1, bei dem man die L-Karnitindehydrogenase vom Genus *Alcaligenes* erhalten hat.

3. Verfahren nach Anspruch 2, bei dem man die L-Karnitindehydrogenase von *Alcaligenes* sp. No. 981 FERM BP-2570 erhalten hat.

4. Verfahren nach einem der vorhergehenden Ansprüche, bei dem die NAD-Gruppe Nicotinamid-adenin-dinucleotid (NAD), Acetylpyridin-adenin-dinucleotid (Acetyl-NAD), Acetylpyridinhypoxanthin-dinucleotid oder Nicotinamid-hypoxanthin-dinucleotid (Deamino-NAD) ist.

5. Verfahren nach einem der vorhergehenden Ansprüche, bei der die Thio-NAD-Gruppe Thionicotinamid-adenin-dinucleotid (Thio-NAD) oder Thionicotinamid-hypoxanthin-dinucleotid ist.

6. Zusammensetzung für die Bestimmung von L-Karnitin, wobei sie die Komponenten 1 bis 3 gemäß einem der vorhergehenden Ansprüche umfaßt.

## Revendications

1. Méthode de dosage de la L-carnitine qui comprend la réaction d'un spécimen comprenant la L-carnitine à doser avec des réactifs comprenant :
1) la L-carnitine déshydrogénase qui a des coenzymes du groupe thionicotinamide adénine dinucléotide (que l'on désignera ci-après par groupe thio-NAD) et du groupe nicotinamide adénine dinucléotide (que l'on désignera ci-après par groupe NAD) et qui catalyse une réaction réversible formant essentiellement la déshydrocarnitine à partir de L-carnitine;
2) A₁ ; et
3) B₂ ;
pour accomplir la réaction de cycle : 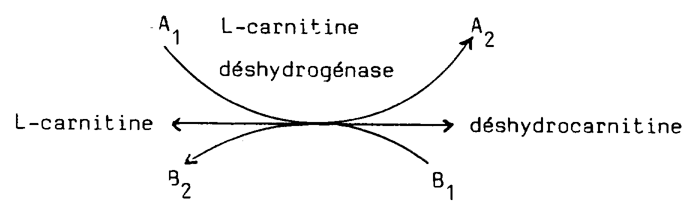 où :
A₁ est le groupe thio-NAD ou le groupe NAD ;
A₂ est la forme réduite de A₁ ;
quand A₁ est le groupe thio-NAD, B₁ est un groupe NAD réduit et quand A₁ est le groupe NAD, B₁ est un groupe thio-NAD réduit et
B₂ est une forme oxydée de B₁ ;
et la mesure de la quantité de A₂ produit ou de B₁ consommé.

2. Méthode selon la revendication 1 où la L-carnitine déshydrogénase a été obtenue à partir du genre Alcaligenes.

3. Méthode selon la revendication 2, où la L-carnitine déshydrogénase a été obtenue de Alcaligenes sp. N° 981 FERM BP-2570.

4. Méthode selon l'une quelconque des revendications précédentes, où le groupe NAD est le nicotinamide adénine dinucléotide (NAD), l'acétylpyridine adénine dinucléotide (acétyl NAD), l'acétylpyridine hypoxanthine dinucléotide ou le nicotinamide hypoxanthine dinucléotide (désamino NAD).

5. Méthode selon l'une quelconque des revendications précédentes, où le groupe thio-NAD est thionicotinamide adénine dinucléotide (thio-NAD) ou thionicotinamide hypoxanthine dinucléotide.

6. Composition appropriée au dosage de la L-carnitine qui comprend les composants 1-3 tels que définis selon l'une quelconque des revendications précédentes.
